# EUROPEAN PATENT APPLICATION

(11) **EP 4 552 587 A1**
(43) Date of publication of application: **14.05.2025**
(21) Application number: 24210855.3
(22) Date of filing: 05.11.2024
(51) Int. Cl.: A61B 17/04

(54) **ALL-SUTURE KNOTLESS REPAIR CONSTRUCT**

(30) Priority: 08.11.2023 US 202363596978 P
(71) Applicant: Smith & Nephew Asia Pacific Pte. Limited, Singapore 138565 (SG); Smith & Nephew Orthopaedics AG, 6300 Zug (CH); Smith & Nephew, Inc., Memphis, TN 38116 (US)
(72) Inventor: Paul, Alyssa, Mansfield, 02048 (US); Shah, Jay Anil, Mansfield, 02048 (US); MacCready, Christopher David, Mansfield, 02048 (US); Patel, Nehal N., Mansfield, 02048 (US)
(74) Representative: Appleyard Lees IP LLP

(57) **Abstract**

An all-suture tissue repair construct including an anchor formed of soft flexible material, a repair suture fixedly coupled thereto and a snare operatively coupled to the repair suture. The tissue repair construct may be provided housed within a suture passer.

## Description

### CROSS-REFERENCE TO RELATED APPLICATIONS

This application claims the benefit to U.S. Provisional App. No. 63/596,978, filed November 8, 2023, titled "All-Suture Knotless Meniscal Repair Construct", herein incorporated by reference, in its entirety

### FIELD

The present disclosure relates to constructs and associated methods for repairing soft tissue. More specifically, this disclosure relates to an all-suture construct that may repair breaks in soft tissue, without the need to tie a knot.

### BACKGROUND

Many orthopedic surgeries involve the use of suture constructs in procedures for repairing tissues, such as repairing breaks in soft tissue such as ligaments, the hip labrum and meniscal tissue. Other procedures may include closing or re-opposing cuts made through soft tissues to provide an opening to gain access to the joint - for example the hip or shoulder capsule may be cut to access the underlying tissues. Preferably repairs are performed arthroscopically.

With arthroscopic surgeries, tying knots in these suture constructs may be challenging and provide inconsistencies. Disclosed herein are various constructs that avoid the need to tie knots in a suture construct during tissue repair.

For meniscal tissue, there are three approaches or categories; an inside-out approach, and outside-in approach and an all-inside approach. The label is generally described by the direction in which the needle and suture are passed through the meniscus, and then secured in place. In general, the all-inside approach is considered preferable, but technically more difficult. Existing systems and associated techniques may place rigid anchors on the outer surface of the capsule side of the meniscus. Other existing systems and techniques may require the need to tie knots. Therefore, there is a need for an all-inside approach that repairs breaks in meniscal tissue that does not require knot tying or requires rigid anchors.

### DEFINITIONS

Described herein are tissue repair systems, which use a soft anchor. The tissue repair systems of this disclosure may provide a high fixation strength, fixing the soft anchor within bone. The tissue repair systems are provided with a locking passage pre-formed, avoiding the need for the surgeon to tie a knot or form the locking passage during the repair. The tissue repair systems may include at least one suture. The term "suture" may include traditional sutures, that may be either hollow or may include braids along their core, unless were specified. The term "suture" may include equivalent flexible members, such as but not limited to suture tape or flattened suture and may in some cases be cable, ribbon or wire, where appropriate.

"Break" is intended to mean an opening, tear or cut in soft tissue. The tissue repair systems disclosed herein are configured to oppose two sides of the opening, tear or cut as part of the repair. Tear may be as a result of injury. A cut may be an incision formed as part of the surgical procedure.

"Soft Anchor" is intended to mean a flexible and/or deformable anchor, where the anchor body is formed of soft, flexible suture-like material. The soft anchor changes to a more laterally and/or radially expanded configuration upon setting or deployment. A tensioning member that is operatively coupled through a portion of the soft anchor may be tensioned to laterally expand the soft anchor. The term "soft anchor" does not preclude it from including supplemental portions that are rigid. In some embodiments, the soft anchor is formed entirely of braided strands. Some soft anchors deform to a deployed configuration that also includes a longitudinal contraction.

"Deploy" is intended to mean to change the shape of the body of the soft anchor so that it is set, fixed or anchored with/within a tissue. Deploying may increase an outer lateral dimension of the anchor body to secure it with a tissue. For example, this may fix the soft anchor within a bone hole. To deploy an anchor changes the anchor to a deployed configuration.

"Lock" or "locked configuration" with respect to a suture construct is intended to mean locking a suture such that the suture may no longer slide in at least one direction. Sliding in this at least one direction for example may loosen a repair tissue that is secured in place. The suture may form a loop including a tissue coupled thereto and the loop perimeter is prevented from sliding and increasing in perimeter size. With respect to an anchor body, a locked configuration is intended to mean locking the anchor body in a deployed configuration to inhibit the anchor body from relaxing/moving out of the deployed configuration.

"Knotlessly locking" or a word stemming derivative therefrom such as knotless locking or knotlessly locked, for example, is intended to mean a lock in a surgical construct or anchor system formed without having the tie a knot. A system provided with a pre-formed knot may be defined as knotlessly locking. Knotlessly locking may be achieved by passing at least one suture along a tortuous route through small openings, or through a suture locking passage construct, may also be called Chinese finger traps, finger cinches or locking splices for example. To knotlessly lock the system, some of the sutures may extend through the suture locking passage of either the same suture or another suture, to form a self-locking adjustable suture construct as described herein. Suture locking passage may be selectively elongated, by applying tension to the locking passage to cinch around the suture disposed therein, thereby locking a portion of the adjustable suture construct.

"Deploying suture" is intended to mean an elongate flexible member that may be a suture(s) (or equivalent as defined herein) that deploys the soft anchor body, usually upon tension being applied to the deploying member. In some embodiments, the deploying member may also provide other functions.

"Repair Suture" is the suture (or equivalent as defined herein) passed through the repair tissue and used to affix the repair tissue to bone.

### SUMMARY

Described herein are various improved methods and devices for tissue repair with a preformed knotlessly locking construct, a stop member and may include a soft anchor.

Disclosed herein is a tissue repair system for repairing a break in a soft tissue. The system may include a repair construct with an anchor, a repair suture, and a snare construct. The anchor may be formed of soft flexible material and is configured to directly engage a first external surface of the soft tissue. The repair suture may have a first end fixedly coupled directly to the anchor with a second end extending from the anchor. The repair suture may include a lumen therealong. The snare construct may be a separate suture from the repair suture and may extend within and along a length of the repair suture lumen in a region of the repair suture directly adjacent and external to the anchor, defining a knotless locking region. The snare construct may extend from both ends of the knotless locking region such that a first end extends from a first end of the knotlessly locking region, and a second end extends from a second end of the knotlessly locking region. The first end of the snare and knotless locking region may be directly adjacent to the anchor than the corresponding second ends. The snare construct first end may be configured to pass through a first side of the break and then couple to the repair member second end. The snare construct first end may include a snare loop. Tension on the snare construct second end when the snare first end is coupled to the repair suture may draw the repair suture second end through the soft tissue in a first direction from a second external surface to the first external surface on the first side of the break, and then draw it in an opposing direction from the first external surface through the knotlessly locking region and through the second external surface on a second side of the break. This may form a repair suture repair loop that extends through the soft tissue and around the break. Tension on the repair suture second end when the loop is formed may actuate the knotless locking region and knotlessly lock the repair loop.

In some embodiments, the repair suture second end may be configured to pass through a second side of the break before coupling to the snare construct first end, such that the repair loop includes three passes through the soft tissue. In some example embodiments the anchor forms a V-shape or a Z-shape and tension on the repair suture may flatten the V-shape or Z-shape against the first external surface. In some example embodiments the knotlessly locking region may have a length that extends through an entire thickness of the soft tissue, through both the first and second external surfaces. In some example embodiments the knotlessly locking region may also extend through an entire thickness of the soft tissue and across the break. In some example embodiments the repair suture first end may form a loop through the anchor, terminating in a fixed knot, to fixedly couple the repair suture to the anchor. In some example embodiments the repair suture second end is spliced with the transfer suture, at a location spaced away from the locking passage region. In some example embodiments the tissue repair system may include an insertion device for arthroscopically inserting the repair construct around the break, the insertion device having a distal end with an axially slideable needle. The needle may pass the snare construct first end through the soft tissue on a first side of the break and is also pass the repair suture second end through the soft tissue on a second side of the break. In some example embodiments the needle may also place the anchor onto the first external surface of the soft tissue while passing either the snare construct first end or the repair suture second end through the soft tissue. The snare construct first end may extend from the knotless locking region first end and then through the anchor to increase a resultant friction of the repair loop. The snare construct may extend from a first external surface of the anchor to an opposing external surface of the anchor. The snare construct may extend through the anchor for a plurality of passes.

Another example embodiment of a tissue repair system for repairing a break in a soft tissue is disclosed. The system may include a repair construct with an anchor, a repair suture, and a snare construct. The anchor may be formed of soft flexible material. The anchor may be configured to directly engage a first external surface of the soft tissue. The repair suture may be fixedly coupled to the anchor at a first end with a second end extending from the anchor. The repair suture may define a lumen. The snare construct may extend within and along a length of the repair suture lumen in a region of the repair suture immediately extending from the anchor, defining a knotless locking region. The snare construct may have a first end extending from a first end of the knotlessly locking region and a second end extending from a second end of the knotlessly locking region. The snare construct first end may be configured to be passed from the first external surface of the tissue, through the soft tissue and through a second external surface of the tissue at a first location and then couple to the repair member second end. Tension on the snare construct second end then draws the repair member second end through the soft tissue in a first direction from the second external surface to the first external surface at the first location, and then from the first external surface through the knotlessly locking region to the second external surface at a second location of the soft tissue, to form a repair loop with the repair suture that extends through the soft tissue. Tension on the repair suture second end is configured to actuate the knotless locking region and knotlessly lock the repair loop.

In some example embodiments the repair suture second end may be configured to be passed through the soft tissue at the second location before coupling to the snare construct first end, such that the repair loop includes three passes through the soft tissue. In some example embodiments the anchor includes at least one bend as provided, defining a V-shape or a Z-shape and tension on the repair suture is configured to flatten the V-shape or Z-shape against itself and the first external surface. In some example embodiments the knotlessly locking region may have a length that extends through an entire thickness of the soft tissue. In some example embodiments the repair suture first end may form a loop through the anchor, terminating in a fixed knot, to fix to the anchor. In some example embodiments the repair suture second end may be to the transfer suture, at a location spaced away from the locking passage region. In some example embodiments the system may include an insertion device having a distal end with an axially slideable needle, and wherein the needle may be configured to pass the snare construct first end through the soft tissue and is also configured to pass the repair suture second end through the soft tissue.

An example method of tissue repair is also disclosed herein. The method may comprise placing an anchor against a first external surface of a soft tissue. The anchor may be a soft anchor and may be coupled to a repair suture that is fixedly coupled to the anchor. A shuttling suture may slidingly extend through a lumen of the repair suture, defining a knotless locking region. The method may also include passing an end of the repair suture through the soft tissue on a first side of the break, and then attaching the end of the repair suture to a first end of the shuttling suture. An opposite end of the shuttling suture may then be tensioned to pull the end of the repair suture through the soft tissue on the first side of the break, through the soft tissue on a second side of the break and also through the locking passage region, such that the repair suture passes through the soft tissue at least 3 times to form a knotlessly locking repair loop.

In some example methods the opposite end of the shuttling suture may also be passed through the soft tissue on the second side of the break before attaching the end of the repair suture to the first end of the shuttling suture. Passing the end of the repair suture through the soft tissue on a first side of the break may further comprise passing the end from the first external surface of the soft tissue through the soft tissue and through a second external surface of the soft tissue. Passing the end of the repair suture through the soft tissue on a first side of the break may concomitantly place the anchor against the first external surface. Passing the end of the repair suture through the soft tissue may comprise placing the knotless locking region within the tissue.

These and other features and advantages will be apparent from a reading of the following detailed description and a review of the associated drawings. It is to be understood that both the foregoing general description and the following detailed description are explanatory only and are not restrictive of aspects as claimed.

### BRIEF DESCRIPTION OF THE DRAWINGS

The disclosure will be more fully understood by reference to the detailed description, in conjunction with the following figures, wherein:
FIG. 1A schematically shows an example all-suture knotless repair construct, in a first configuration, in accordance with this disclosure;
FIG. 1B schematically shows an example all-suture knotless repair construct, with the repair loop formed, in accordance with this disclosure;
FIG. 2A shows an example all-suture knotless repair construct, in accordance with this disclosure;
FIG. 2B shows an example all-suture knotless repair construct, in accordance with this disclosure;
FIGS. 3A-3J illustrate various views of a method of soft tissue repair with an all-suture knotless repair construct, accordance with this disclosure;
FIG. 4 illustrates a suture passer with an all-suture knotless repair construct assembled thereto;
FIG. 5A schematically shows another example all-suture knotless repair construct, in a first configuration, in accordance with this disclosure; and
FIG. 5B schematically shows the other example all-suture knotless repair construct, with the repair loop formed, in accordance with this disclosure.

### DETAILED DESCRIPTION

In the description that follows, like components have been given the same reference numerals, regardless of whether they are shown in different examples. To illustrate example(s) in a clear and concise manner, the drawings may not necessarily be to scale and certain features may be shown in somewhat schematic form. Features that are described and/or illustrated with respect to one example may be used in the same way or in a similar way in one or more other examples and/or in combination with or instead of the features of the other examples.

As used in the specification and claims, for the purposes of describing and defining the invention, the terms "about" and "substantially" are used to represent the inherent degree of uncertainty that may be attributed to any quantitative comparison, value, measurement, or other representation. The terms "about" and "substantially" are also used herein to represent the degree by which a quantitative representation may vary from a stated reference without resulting in a change in the basic function of the subject matter at issue. "Comprise," "include," and/or plural forms of each are open ended and include the listed parts and can include additional parts that are not listed. "And/or" is open-ended and includes one or more of the listed parts and combinations of the listed parts. Use of the terms "upper," "lower," "upwards," and the like is intended only to help in the clear description of the present disclosure and are not intended to limit the structure, positioning and/or operation of the disclosure in any manner.

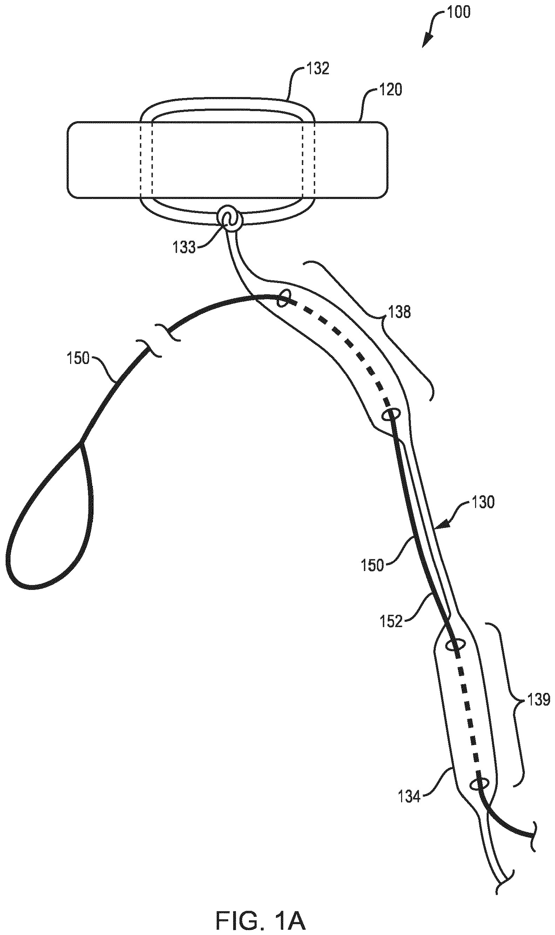

FIG. 1A schematically shows an all-suture construct 100 that may repair soft tissue, in accordance with this disclosure. This all-suture construct 100 may repair a tear in meniscal tissue of the knee. This all-suture construct 100 may repair a cut made through a hip capsule. FIG. 1A illustrates construct 100 in a first configuration, which may be the configuration that is provided, and/or as pre-assembled to an insertion device. Construct 100 may include a soft anchor 120, a repair suture 130 and a transfer or shuttling suture 150. Soft anchor 120 may be a tubular body formed of braided threads, similar to anchor disclosed in at least U.S. Patent 9,962,149, titled "Tissue Repair Assembly"; commonly owned and herein incorporated by reference in its entirety. Anchor 120 may be a flexible member that forms at least one bend similar to anchor disclosed in U.S. Patent 8,795,334, titled "Tissue Repair"; commonly owned and herein incorporated by reference in its entirety. Soft anchor 120 may be 0.25 inches (6mm) long. Soft anchor 120 may be formed of #5 DuraBraid that is heat cut at both ends to bind the ends.

Repair suture 130 may include a lumen therealong. Repair suture 130 may be fixedly coupled to anchor 120. As shown repair suture 130 may form a loop 132 around anchor 120, the loop 132 closed with a knot 133. Knot 133 may be a fixed knot 133, in that it does not slide but is fixed in location along the repair suture 130, fixing the circumferential length of the loop 132. In some embodiments the repair suture is spliced through itself instead of a knot 133, to form a splice loop through the anchor 120. In other embodiments, repair suture may be fixedly coupled via other fixing means such as welding or adhesives that may supplement or replace the knotted loop 132.

Transfer suture 150 may be a separately formed suture, that may absent a lumen. Transfer suture 150 may couple to the repair suture 130 via the repair suture lumen. Transfer suture 150 may be provided extending along repair suture lumen, defining a locking passage region 138. Transfer suture 150 is configured to draw a repair suture end 134 around through tissue and through locking passage region 138. Transfer suture 150 may be configured to draw repair suture end 134 around a tissue break. Transfer suture 150 may extend along the repair suture lumen a second time, defining a splice end 139. Splice end 139 may preferably manage the two suture ends and reduce complexity of the system. This construct may preferably be inserted and passed with a mattress style system, such that only two passes through tissue are preferable. Linking the suture ends via spice 139 allows for an insertion instrument to pass both transfer and repair suture together in a single pass reliably, defining one of the two passes.

Transfer suture 150 may be absent a lumen and may define a smaller diameter suture than the repair suture. In some embodiments transfer suture 150 may be a flexible member such as a cable or a wire. Transfer suture 150 may include a looped end 154 for receiving the repair suture end. Looped end 154 may be a fixed loop in that it does not reduce with tension. In other embodiments, looped end 154 may include a reducing loop, that reduces and cinches around the repair suture end with tension.

Knotless locking region 138 may be preferably directly adjacent the anchor 120. This may help keep the repair loop 160 as close to the anchor 120 as possible and reduce slack in the final repair. In some embodiments the transfer suture 150 may pierce the knot 133 immediately before entering the repair suture lumen. In some embodiments, the transfer suture 150 may punch through the entire thickness of the repair suture and immediately enter the repair suture lumen. The knotless locking region may have a first end defined as where the first end of the transfer suture exits, and a second end defined as where the second end 152 of the transfer suture exits.

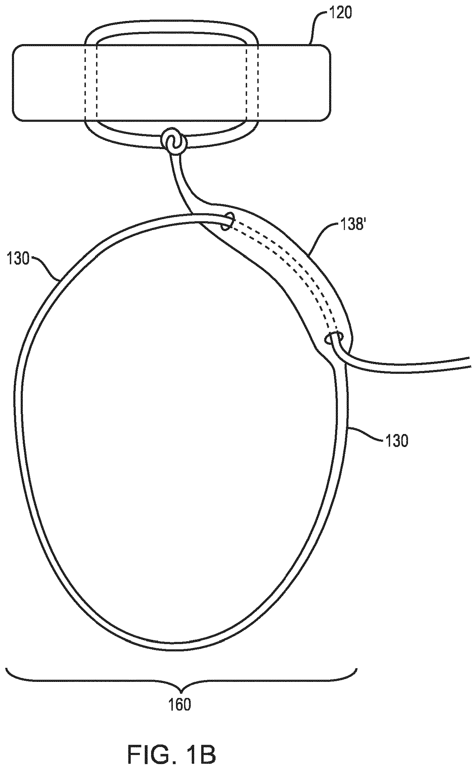

FIG. 1B schematically shows the all-suture construct 100 with the repair loop 160 formed. Transfer suture 150 has been coupled to repair suture end 134 and tension on the transfer suture end 152 slides the transfer suture 150 and transfers the repair suture end 134 through or around tissue and also through the knotless locking region 138, to form the knotless locking passage 138'.

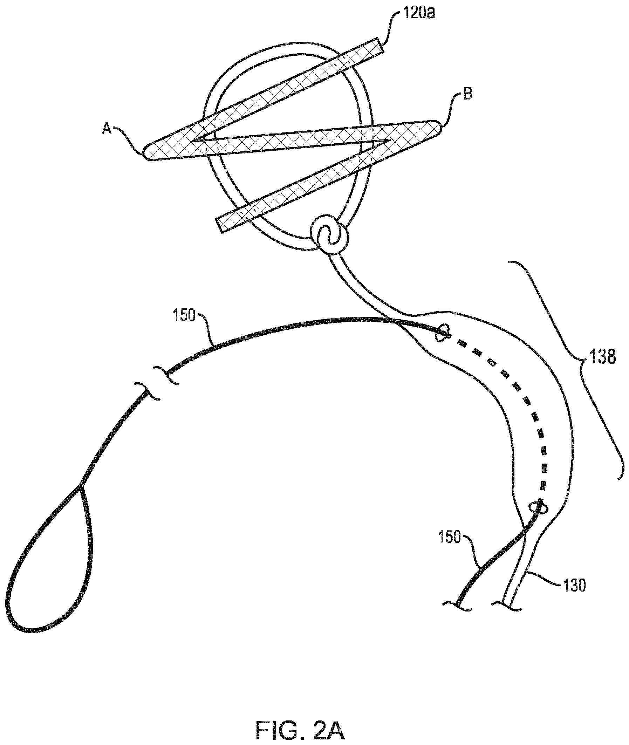

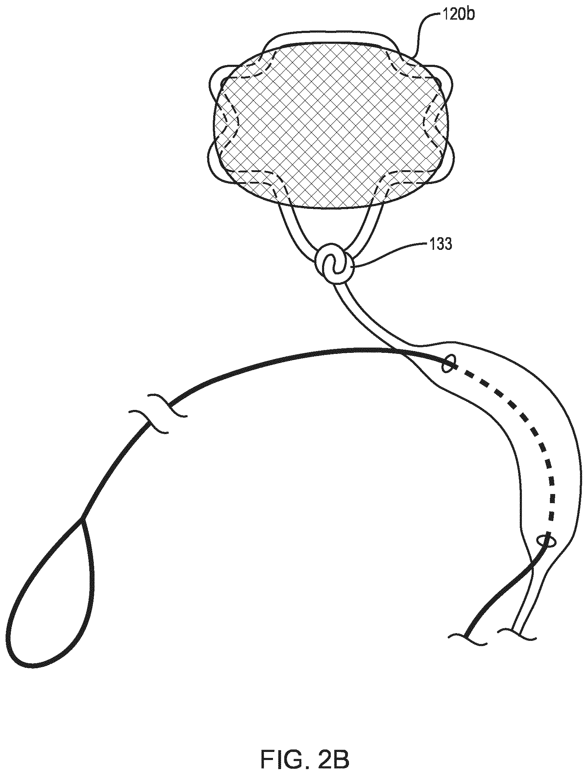

FIG. 2A illustrates construct 100, with a zig-zag style soft anchor 120a. As shown, anchor 120a may forms two bends (A, B) defining 3 legs - forming a Z shape. In other embodiments a single bend and two legs may only be needed, forming a V shape. As shown, each leg is distinguishable in the figures. However, upon tension being applied to the construct 100, anchor 120a may compress or flatten further, reducing the anchor height or prominence. This is preferable as it will lie flatter on the tissue external surface, reducing any agitation with adjacent tissues. FIG. 2B illustrates all-suture construct 100, with a tubular body style soft anchor 120b.

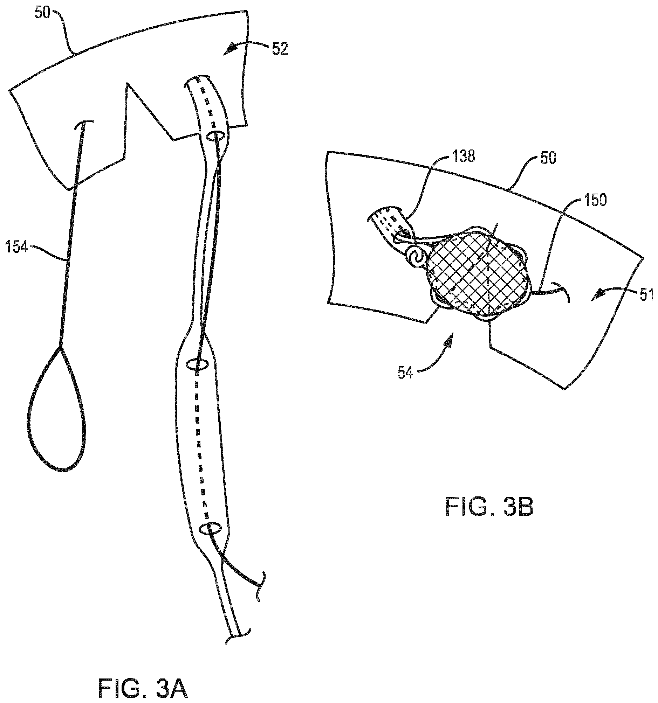

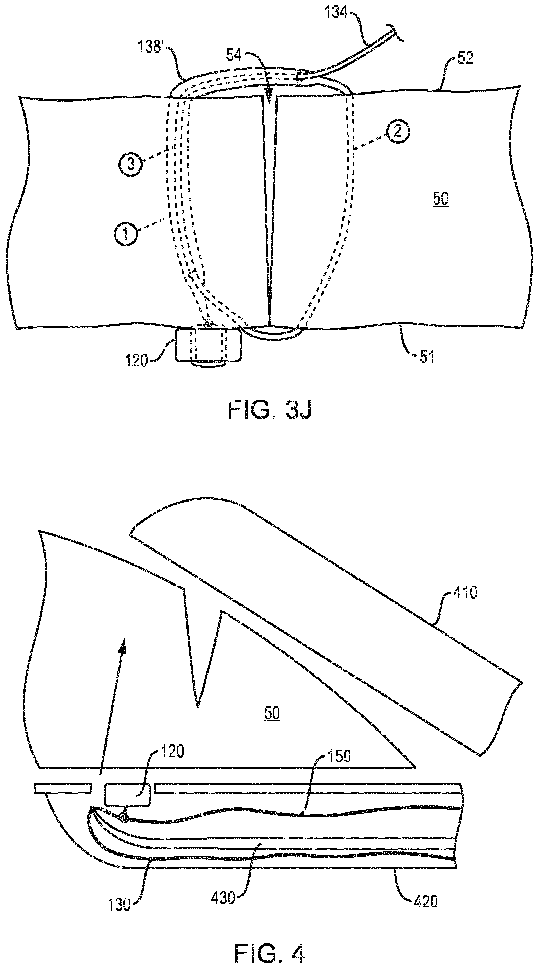

FIG. 3A-3J illustrates a method of meniscal tear repair with construct 100. Similar methods may repair two leaflets of a hip labrum. All-suture construct 100 may be provided loaded in an arthroscopic suture passer 400 with a pair of jaws at a distal end of the passer 400, as shown in FIG. 4. Arthroscopic suture passer may preferably make two passes through a tissue. Anchor 120 may be placed on an inferior external surface 51 of the knee meniscus 50 adjacent and to one side of the break 54. The passer 400 may include a needle 430 that may pass the repair suture end 134 and transfer suture end 152 simultaneously through from the inferior external surface 51 to the superior external surface 52. Repair suture end 134 and transfer suture end 152 may be spliced together. Passing the repair suture end 134 and transfer suture end 152 simultaneously may concomitantly place the locking passage region 138 within the tissue 50. After passing, the locking passage region 138 may be at least partially within tissue 50 and may also extend from superior external surface 52.

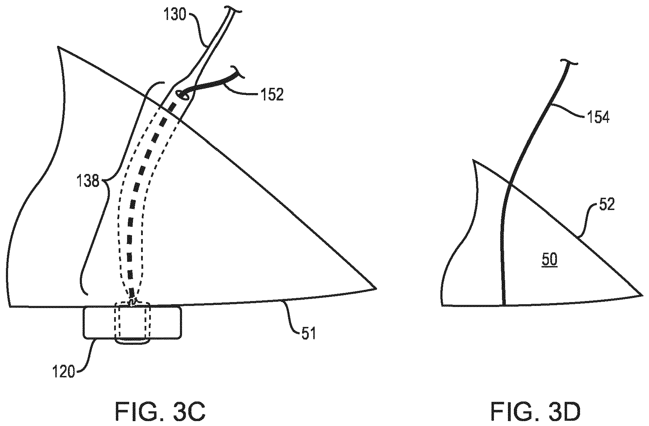

The suture passer 400 may then be moved to the other side of the break 54 and the needle 430 that may then pass the second transfer suture end 154 through from the inferior external surface 51 to the superior external surface 52. FIG. 3A illustrates a superior side view showing both the repair suture 134 and transfer suture 150 extending through and from the external superior side surface 52 on one side of the break 54, with the locking passage region 138 extending through the superior external surface. FIG. 3A illustrates a superior side view showing transfer suture 150 extending through and from the external superior side surface 52 on the other side of the break 54. FIG. 3B illustrates an inferior side view showing both the anchor 120 (example anchor 120b shown), with the locking passage 138 and transfer suture 150 extending through the inferior surface 51 of the tissue 50. FIG. 3C illustrates a simplified cross section view of the pass that includes passing the repair suture end 134 and transfer suture end 152 simultaneously through from the inferior external surface 51 to the superior external surface 52. FIG. 3D illustrates a simplified cross section view of the pass that includes passing the transfer suture end 154 through from the inferior external surface 51 to the superior external surface 52.

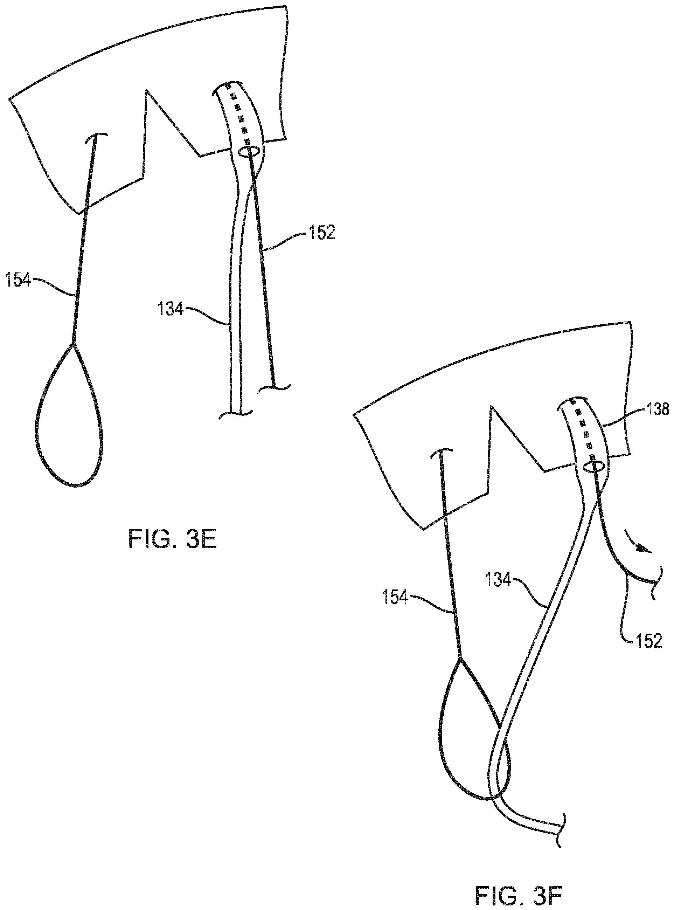

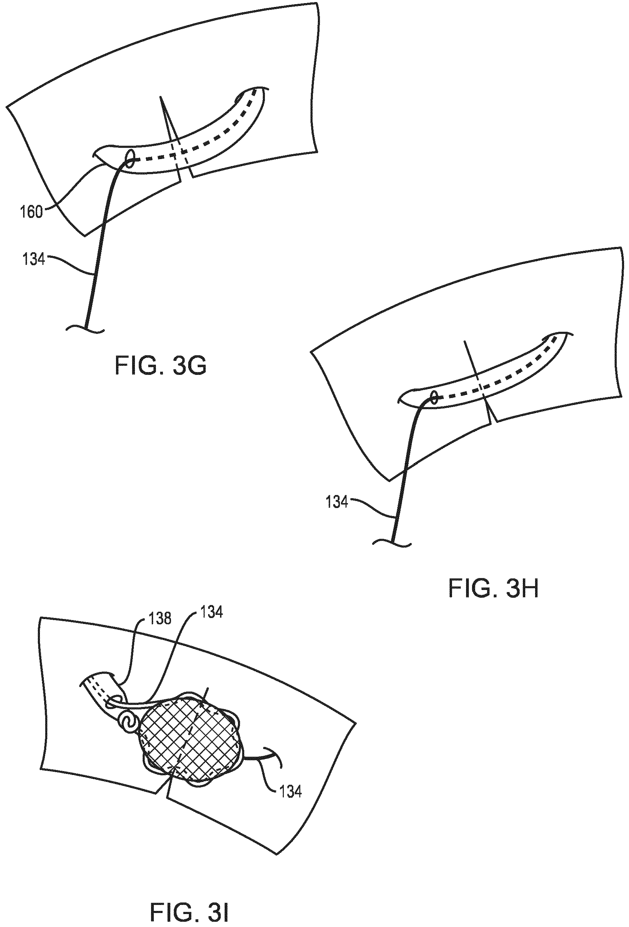

FIG. 3E illustrates a superior side view with a subsequent step including disconnecting suture ends 152 and 134, if connected via splice 139. FIG. 3F illustrates the superior side view with a subsequent step including snaring repair suture end 134 with transfer suture loop at end 154 and then drawing transfer suture end 152 (shown with arrow) so as to transfer or shuttle repair suture end 134 along the transfer suture path through the tissue. This includes first drawing the repair suture end 134 through from the superior external surface 52 to the inferior external surface 51, across the break 54 and then back through the tissue from the inferior external surface 51 to the superior external surface 52. The repair loop 160 is now formed, with the repair suture being passed three times through the tissue. As shown in FIG. 3G, the locking passage region 138 now includes the repair suture 130 and may extend both through the tissue 50 and across the external superior surface of the tissue 50. Pulling tension on end 152 once the repair loop 160 may approximate the break 54 and then cinch the locking passage (FIG. 3H). The locking passage in the cinched form now prevents the repair suture 130 from slipping and the repair loop 160 from loosening. FIG. 3I illustrates an inferior side view with the anchor 120 and repair suture end 134.

FIG. 3J is a simplified cross section showing loop 160, including a first pass, second and third pass of repair suture 130 through the tissue (indicated with corresponding numbers). Anchor 120 abuts inferior surface 51. Locking passage 138 extends through the tissue 50 and may also extend across the superior surface 52.

FIG. 4 illustrates an example distal end of a device 400, that may be provided preassembled with construct 100. A simplified schematic of construct 100 is shown within lower jaw 420, for simplification of the figure. Device may include two jaws, 410, 420, that are moveable relative to each other. Lower jaw 420 may house a needle 430 that may axially move and pierce the tissue 50 (see arrow). Needle may include a hook or engaging means to engage sequentially two portions of the construct 100. For example, needle may be provided engaged to the repair suture 130 and transfer suture 150, so that actuation of the needle 430 may concomitantly pass the repair suture 130, transfer suture 150 and knotless locking region 138 through tissue 50. Tension on the repair suture 130 during this first pass may draw the all-suture anchor 120 out of or away from lower jaw 420 and place it on the inferior surface of tissue 50. Tension on the repair suture 130 during this first pass may laterally expand all-suture anchor increase engagement between the anchor 120 and tissue external surface. Tension on the repair suture 130 during this first pass may flatten or shorten an all-suture anchor height, reducing its prominence away from the tissue external surface, which may reduce any irritation of the local tissues. Retraction of the needle 430 back into the lower jaw 430 may then capture the transfer suture 150 only. The device may then be moved to a different side of the break and the needle 430 advanced to pass the transfer suture 150 through the tissue 50. Device 400 may be similar to the devices disclosed in US Patents 9,861,354; 10,188,382; 10,004,492; 10441273; 10,524,779 and 11,202,623, commonly owned and herein incorporated by reference in its entirety.

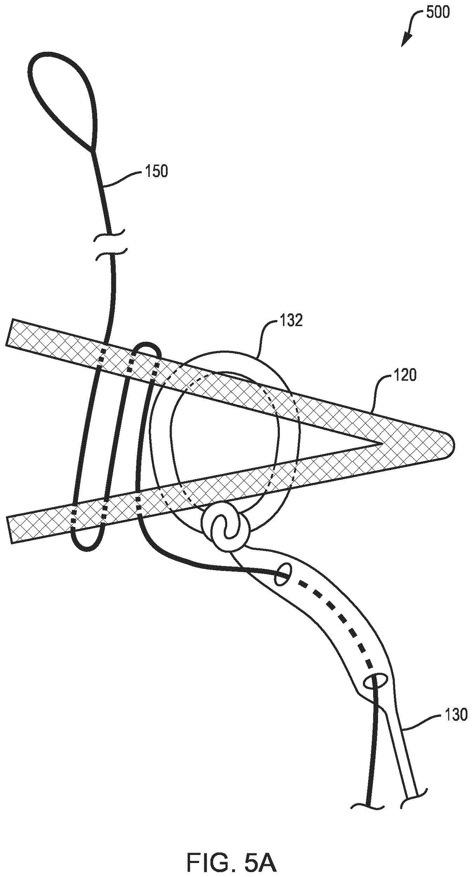

FIG. 5A schematically shows another example all-suture construct 500 that may repair soft tissue, in accordance with this disclosure. Like elements are given the same identifier disclosed in FIG. 1A. Construct 500 is similar to construct 100, except where noted. This all-suture construct 500 may repair a break in soft tissue. FIG. 5A illustrates construct 500 in a first configuration, which may be the configuration that is provided, and/or as pre-assembled to an insertion device. Construct 500 may include a soft anchor 120, a repair suture 130 and a transfer or shuttling suture 150. Soft anchor 120 may be similar to those disclosed herein.

Repair suture 130 may include a lumen therealong. Repair suture 130 may be fixedly coupled to anchor 120. Transfer suture 150 may be interwoven through anchor. Transfer suture 150 may be provided extending along repair suture lumen, defining a locking passage region 138. Transfer suture 150 draws a repair suture end 134 around through tissue and then through the anchor 120 to weave through and around the anchor 120 and also through locking passage region 138. Transfer suture 150 may be configured to draw repair suture end 134 around a tissue break. Transfer suture 150 may extend along the repair suture lumen a second time, defining a splice end 139. Transfer suture 150 may be absent a lumen and may define a smaller diameter suture than the repair suture. In some embodiments transfer suture 150 may be a flexible member such as a cable or a wire. Transfer suture 150 may include a looped end 154 for receiving the repair suture end. Knotless locking region 138 may be preferably directly adjacent the anchor 120. This may help keep the repair loop 160 as close to the anchor 120 as possible and reduce slack in the final repair

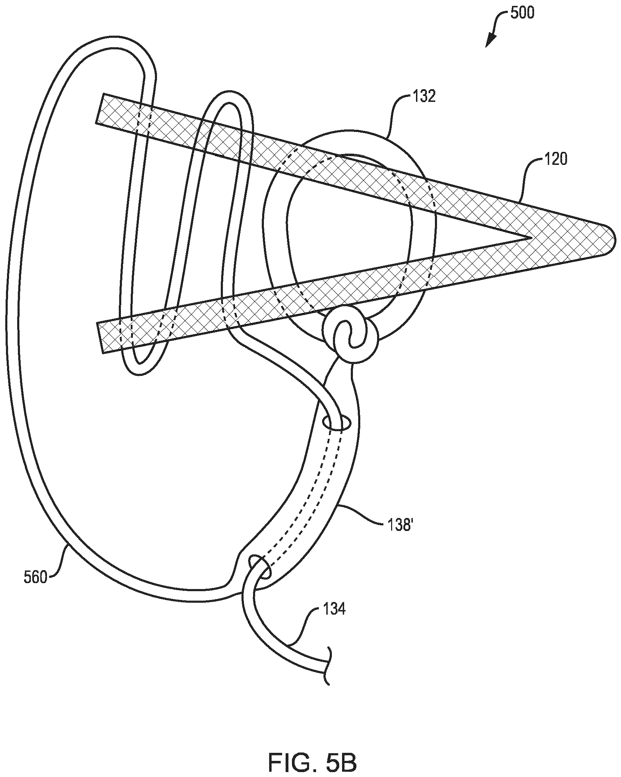

FIG. 5B schematically shows the all-suture construct 500 with the repair loop 560 formed. Transfer suture 150 has been coupled to repair suture end 134 and tension on the transfer 152 end slides the transfer suture 150 and transfers the repair suture end 134 through or around tissue, through and around the anchor 120 and also through the knotless locking region 138, to form the knotless locking portion or passage 138'. Adding this pass through the anchor 120 may add a friction inducing zone to the construct 500 and therefore increase the overall friction of the construct locked loop 560; allowing the knotless locking passage 138' to be shorter but provide a high locking force.

One skilled in the art will realize the disclosure may be embodied in other specific forms without departing from the spirit or essential characteristics thereof. The foregoing examples are therefore to be considered in all respects illustrative rather than limiting of the disclosure described herein. Scope of the disclosure is thus indicated by the appended claims, rather than by the foregoing description, and all changes that come within the meaning and range of equivalency of the claims are therefore intended to be embraced therein.

## Claims

1. A tissue repair system for repairing a break in a soft tissue comprising;
a repair construct comprising:
an anchor formed of soft flexible material;
a repair suture having a first end fixedly coupled to the anchor and a second end extending from the anchor, wherein the repair suture defines a lumen;
a snare construct extending within and along a length of the repair suture lumen in a region of the repair suture immediately adjacent and external to the anchor, defining a knotless locking region, the snare construct having a first end extending from a first end of the knotless locking region and a second end extending from a second end of the knotless locking region;
wherein the snare construct first end is configured to couple to the repair member second end and while coupled, tension on the snare construct second end is configured to draw the repair member second end through the knotless locking region to form a repair loop in the repair suture and wherein tension on the repair suture second end with the repair loop formed is configured to actuate the knotless locking region and knotless lock the repair loop.

2. The tissue repair system of claim 1, wherein the anchor forms a V-shape or a Z-shape and wherein tension on the repair suture in configured to flatten the V-shape or Z-shape.

3. The tissue repair system of claim 1 or 2, wherein the repair suture first end includes a loop extending through the anchor, the loop terminating in a fixed knot that is disposed on an external surface of the anchor and immediately adjacent the knotless locking region, the loop and fixed knot configured to fixedly couple the repair suture to the anchor.

4. The tissue repair system of claim 1, 2, or 3, wherein the repair suture second end is spliced with the snare construct second end, at a location spaced away from the knotless locking region, defining a spliced end configured to provide a single limb for simultaneous passing of both second ends in a single pass.

5. The tissue repair system of claim 4, further comprising an insertion device configured to arthroscopically pass the repair construct around the break, the insertion device having a distal end with an axially slideable needle, and wherein the needle is configured to pass the snare construct first end and also pass the spliced end through the soft tissue.

6. The tissue repair system of claim 5, wherein the insertion device is configured to place the anchor onto a first external surface of the soft tissue while passing either the snare construct first end or the spiced end through the soft tissue.

7. The tissue repair system of any preceding claim, wherein the snare construct first end extends from the knotless locking region first end and then through the anchor to increase a resultant friction of the repair loop.

8. The tissue repair system of claim 7, wherein the snare construct extends from a first external surface of the anchor to an opposing external surface of the anchor.
